# EUROPÄISCHE PATENTANMELDUNG

(11) **EP 4 450 029 A1**
(43) Veröffentlichungstag der Anmeldung: **23.10.2024**
(21) Anmeldenummer: 24170012.9
(22) Anmeldetag: 12.04.2024
(51) Int. Cl.: A61F 2/38, A61B 5/00, A61B 5/01, A61B 5/11, A61B 5/145, A61F 2/48, G01N 33/68, A61B 34/20, A61B 90/00, A61F 2/30

(54) **GELENKENDOPROTHESE, INSBESONDERE KNIEENDOPROTHESE, UND SYSTEM UMFASSEND EINE GELENKENDOPROTHESE**

(30) Priorität: 20.04.2023 DE 102023110011
(71) Anmelder: Aesculap AG, 78532 Tuttlingen (DE)
(72) Erfinder: Bollinger, Arthur, 78532 Tuttlingen (DE); Lenzenhuber, Frederick, 78532 Tuttlingen (DE); Richter, Berna, 78570 Mühlheim (DE)
(74) Vertreter: DREISS Patentanwälte PartG mbB

(57) **Zusammenfassung**

Die Erfindung betrifft eine Gelenkendoprothese (10), insbesondere Knieendoprothese (10), wobei die Gelenkendoprothese (10) zumindest teilweise aus einem Kunststoff, insbesondere Polyetheretherketon (PEEK) oder Polyetheretherketon, (PEEK),-Verbundwerkstoff, gefertigt ist, oder Kunststoff, insbesondere Polyetheretherketon (PEEK) oder Polyetheretherketon, (PEEK),-Verbundwerkstoff, umfasst, wobei die Gelenkendoprothese (10) wenigstens eine Sensoreinrichtung (36), insbesondere Drucksensor, zum Erfassen einer an einer Gleitfläche der Gelenkendoprothese (10) wirkenden Kraft, insbesondere eine Druckkraft, elektronische Mittel (34, 54) zum Erhalten der von der mindestens einen Sensoreinrichtung erfassten Daten und eine Energieversorgungseinrichtung (44, 56) zum Versorgen der wenigstens einen Sensoreinrichtung und der elektronischen Mittel umfasst, und wobei die wenigstens eine Sensoreinrichtung in die Gelenkendoprothese (10), insbesondere in Kunststoff, insbesondere Polyetheretherketon (PEEK) oder Polyetheretherketon, (PEEK),-Verbundwerkstoff, eingebettet ist.

## Beschreibung

Die Erfindung betrifft eine Gelenkendoprothese, insbesondere Knieendoprothese.

Gelenkendoprothesen, insbesondere Knieendoprothesen, sind aus dem Stand der Technik bekannt. Aus dem Stand der Technik sind außerdem Instrumente oder Implantatkomponenten bekannt, die vorübergehend, beispielsweise während der Operation oder vor der Implantation der eigentlichen Prothese in den Gelenkbereich des Patienten implantiert werden. Beispielsweise ist aus WO2021/014313 A1 eine Spacer-Komponente bekannt, die dazu bestimmt ist, vorübergehend in den Gelenkbereich für den Ersatz einer Gelenkprothese und für die Erhaltung der Dimensionen oder Räume des Gelenkbereichs des Patienten vor der Implantation einer neuen Prothese implantiert zu werden. Die Spacer-Komponente umfasst beispielsweise verschiedene Sensoren, die die Erfassung von Daten, beispielsweise einer Temperatur, ermöglichen, während des vorübergehenden Zeitraums.

Der vorliegenden Erfindung liegt die Aufgabe zugrunde, eine Gelenkendoprothese bereitzustellen, die das Ermitteln einer Gelenkbelastung in vivo ermöglicht.

Diese Aufgabe wird gelöst durch eine Gelenkendoprothese mit den Merkmalen des Anspruchs 1. Gemäß Anspruch 1 ist vorgesehen, dass die Gelenkendoprothese zumindest teilweise aus einem Kunststoff, insbesondere Polyetheretherketon (PEEK) oder Polyetheretherketon, (PEEK),-Verbundwerkstoff, gefertigt ist, oder Kunststoff, insbesondere Polyetheretherketon (PEEK) oder Polyetheretherketon, (PEEK),-Verbundwerkstoff, umfasst.

Als Kunststoff wird vorzugsweise ein körperverträglicher Kunststoff gewählt. Vorteilhafterweise wird ein Kunststoff gewählt mit einem sehr ähnlichen beziehungsweise nahezu identischen Elastizitätsmodul, E-Modul, wie das E-Modul von menschlichem Knochen. Beispielweise umfasst PEEK ein solches ähnliches E-Modul. Auf diese Weise können die im Gelenk unter Belastung wirkenden Kräfte unverfälscht an eine Sensoreinrichtung übertragen werden. Dies ist beispielsweise bei metallischen Implantaten nicht ohne weiteres möglich. Das E-Modul von Metall unterscheidet sich stark vom E-Modul des menschlichen Knochens.

Weiter ist vorgesehen, dass die Gelenkendoprothese wenigstens eine Sensoreinrichtung, insbesondere Drucksensor, zum Erfassen einer an einer Gleitfläche der Gelenkendoprothese wirkenden Kraft, insbesondere eine Druckkraft, elektronische Mittel zum Erhalten der von der mindestens einen Sensoreinrichtung erfassten Daten und eine Energieversorgungseinrichtung zum Versorgen der wenigstens einen Sensoreinrichtung und der elektronischen Mittel, umfasst. Bei der Sensoreinrichtung, insbesondere Drucksensor, zum Erfassen der an der Gleitfläche der Gelenkendoprothese, insbesondere unter Belastung, wirkenden Kraft handelt es sich beispielsweise um einen Federkörper-Kraftaufnehmer, wobei einer Verformung des Federkörpers über Dehnungsmessstreifen erfasst wird, oder ein Piezo-Kraftaufnehmer, wobei bei Verformung eines piezoelektrischen Materials eine elektrische Spannung erfasst werden kann.

Die elektronischen Mittel umfassen beispielsweise eine Speichereinrichtung zum Speichern, insbesondere Zwischenspeichern, der von der Sensoreinrichtung erfassten Daten, und/oder eine Datenübertragungseinrichtung zum Übertragen der gespeicherten oder der erfassten Daten. Die elektronischen Mittel umfassen beispielsweise auch elektrischen Verbindungen zwischen der Sensoreinrichtung und/oder der Speichereinrichtung und/oder der Datenübertragungseinrichtung zum Übertragen der erfassten Daten zwischen der Sensoreinrichtung und/oder der Speichereinrichtung und/oder der Datenübertragungseinrichtung. Bei der Energieversorgungseinrichtung handelt es sich beispielsweise um einen, insbesondere aufladbaren, Energiespeicher, beispielsweise eine Batterie oder ein Akkumulator, und gegebenenfalls auch elektrische Verbindungen zum Verbinden des Energiespeichers mit der Sensoreinrichtung.

Erfindungsgemäß ist weiter vorgesehen, dass die wenigstens eine Sensoreinrichtung in die Gelenkendoprothese, insbesondere in Kunststoff, insbesondere Polyetheretherketon (PEEK) oder Polyetheretherketon, (PEEK),-Verbundwerkstoff, eingebettet ist. Beispielsweise ist bei der Gelenkendoprothese eine entsprechende Aussparung vorgesehen, in die Sensoreinrichtung eingesetzt ist. Beispielsweise wird die Aussparung beim Herstellen der Gelenkendoprothese, beispielsweise mittel Spritzguss und/oder 3D-Druck, vorgesehen. Alternativ kann die Sensoreinrichtung bereits beim Spritzgießen oder beim 3D-Drucken der Gelenkendoprothese in die Gelenkendoprothese integriert, sozusagen in den Kunststoff eingegossen und/oder von dem Kunststoff umspritzt, werden.

Auf diese Weise kann die Sensoreinrichtung im Bereich der Gleitfläche der Gelenkendoprothese, an der die wirkende Kraft erfasst werden soll vorgesehen werden. Über das Kunststoffmaterial der Gelenkendoprothese mit dem sehr ähnlichen beziehungsweise nahezu identischen E-Modul, wie das E-Modul von menschlichem Knochen, kann die im Gelenk an der Gleitfläche unter Belastung wirkende Kraft unverfälscht und direkt auf die Sensoreinrichtung übertragen und von der Sensoreinrichtung erfasst werden.

Es kann auch vorteilhaft sein, wenn die Energieversorgungseinrichtung oder Teile der Energieversorgungseinrichtung, und/oder die elektronischen Mittel oder Teile der elektronischen Mittel in der Gelenkendoprothese, insbesondere in Kunststoff, insbesondere Polyetheretherketon (PEEK) oder Polyetheretherketon, (PEEK),-Verbundwerkstoff, eingebettet sind. Beispielsweise sind dafür entsprechende Aussparungen vorgesehen. Beispielsweise können die entsprechenden Teile bereits beim Spritzgießen der Gelenkendoprothese in die Gelenkendoprothese integriert, sozusagen eingegossen, werden.

Gemäß einer vorteilhaften Ausführungsform ist vorgesehen, dass die Energieversorgungseinrichtung einen Energiespeicher und Mittel zum Aufladen des Energiespeichers, insbesondere Mittel zum induktiven Laden, umfasst. Beispielsweise umfassen die Mittel zum induktiven Laden eine Empfangsspule, in die mittels einer außerhalb des Körpers des Patienten in die Nähe bringbare Sendespule eine Spannung induzierbar ist. Auf diese Weise kann der Energiespeicher, insbesondere bedarfsweise geladen werden.

Gemäß einer vorteilhaften Ausführungsform ist vorgesehen, dass die Gelenkendoprothese, insbesondere die elektronischen Mittel der Gelenkendoprothese, eine Datenübertragungseinrichtung umfasst, wobei die Datenübertragungseinrichtung ausgebildet ist, Daten drahtlos, insbesondere an eine externe Empfangseinrichtung zu übertragen. Die drahtlose Datenübertragung erfolgt beispielweise mittels einer Bluetooth basierten oder einer NFC basierten Datenübertragung. Die Datenübertragung erfolgt beispielsweise an ein Mobilfunkgerät.

Die Daten können zu Forschungs- und Wissenschaftszwecken und für die Weiterentwicklung herangezogen werden. Die Daten über die wirkenden Kräfte und gegebenenfalls weitere Daten können zur Weiterentwicklung und zur Ursachenforschung, beispielweise bei Fehlfunktion oder Bruch von Endoprothesen, herangezogen werden.

Gemäß einer bevorzugten Ausführungsform ist die Gelenkendoprothese eine Kniegelenkendoprothese oder bildet zumindest einen Teil einer Kniegelenkendoprothese. Die Gelenkendoprothese ist oder umfasst wenigstens eine erste Gelenkkomponente, beispielsweise eine Femurkomponente oder eine Tibiakomponente oder eine Meniskuskomponente. Die Gelenkendoprothese kann auch eine Femurkomponente, eine Tibiakomponente und eine, insbesondere an die angeformte Tibiakomponente Meniskuskomponente umfassen. Vorteilhafterweise ist wenigstens die Femurkomponente aus Kunststoff, insbesondere PEEK oder einem PEEK-Verbundwerkstoff, gefertigt.

Die erste Gelenkkomponente der Gelenkendoprothese ist beispielsweise eine Femurkomponente, wobei die Femurkomponente wenigstens eine Femurkondyle umfasst. In einem distalen Bereich der Femurkondyle ist wenigstens eine Sensoreinrichtung zum Erfassen einer an einer Gleitfläche einer Gelenkendoprothese wirkenden Kraft vorgesehen. Die Femurkondyle umfasst eine Femurgelenkfläche, wobei die Femurgelenkfläche eine Gleitfläche der Gelenkendoprothese bildet und mittels der Sensoreinrichtung die an der Femurgelenkfläche in dem distalen Bereich der Femurkondyle wirkende Kraft erfassbar ist. Bei einem einseitigen Oberflächenersatz umfasst die Femurkomponente entweder eine laterale oder einer mediale Femurkondyle. Bei einem kompletten Oberflächenersatz umfasst die Femurkomponente entweder eine laterale oder einer mediale Femurkondyle. In diesem Fall ist beispielsweise in einer jeweiligen Femurkondyle in einem jeweiligen distalen Bereich der Femurkondyle wenigstens eine Sensoreinrichtung vorgesehen. Im Kniegelenk erfolgt die tibiofemorale Kraftübertragung über die mediale und laterale Femurkondyle. Durch beidseitiges Vorsehen der Sensoren können die wirkenden Kräfte separat an einer jeweiligen Kondyle erfasst werden.

Die Femurkomponente umfasst beispielweise eine retropatellare Gleitrinne, Trochlea. Es kann vorteilhaft sein, wenn wenigstens ein Teil der Energieversorgungseinrichtung, beispielsweise eine Empfangsspule zum induktiven Laden, im Bereich der Trochlea vorgesehen ist. Auf diese Weise kann eine außerhalb des Körpers des Patienten in die Nähe bringbare Sendespule vergleichsweise nah Empfangsspule herangebracht werden.

Die erste Gelenkkomponente der Gelenkendoprothese ist beispielsweise eine Femurkomponente, wobei die Femurkomponente wenigstens eine Femurkondyle und/oder Trochlea umfasst. Vorteilhafterweise kann eine Sensoreinrichtung oder eine weitere Sensoreinrichtung zum Erfassen einer an einer Gleitfläche der Gelenkendoprothese wirkenden Kraft in einem posterioren Bereich der Femurkondyle vorgesehen sein, und/oder eine Sensoreinrichtung oder eine weitere Sensoreinrichtung zum Erfassen einer an einer Gleitfläche der Gelenkendoprothese wirkenden Kraft im Bereich der Trochlea vorgesehen sein. Im posterioren Bereich der Kondylen wirken bei einem Patienten beim Beugen der Kniegelenke, beispielsweise beim Treppensteigen, relativ große Kräfte an den Femurgelenkflächen. Im Bereich der Trochlea kann eine Kraft gemessen werden, die an der Fläche zwischen Trochlea und Patella wirkt, auch Patelladruck genannt. Werte des Patalladrucks können ein Indikator für eine korrekte oder inkorrekte Positionierung der Endoprothese sein. Grundsätzlich ist der Patelladruck bzw. der Druckverlauf auch heute noch ein wenig erforschtes Gebiet. Die erfassten Daten können für Forschung und Wissenschaft bedeutende Erkenntnisse liefern. Beispielsweise können die Daten zum Verbessern von Gelenkendoprothese herangezogen werden.

Es kann vorgesehen sein, dass die Gelenkendoprothese wenigstens eine Sensoreinrichtung zum Detektieren einer Infektion in der Gelenkendoprothese und/oder in einem Implantationsbereich der Gelenkendoprothese, insbesondere mittels Erfassen von C-Reaktivem Protein, und/oder wenigstens eine Sensoreinrichtung zum Erfassen einer Temperatur, und/oder wenigstens eine Sensoreinrichtung zum Erfassen einer Beschleunigung umfasst. Mittels eines CRP-Sensors kann beispielsweise das Vorhandensein bzw. eine Konzentration von C-reaktiven Protein erfasst werden. C-reaktives Protein kann ein Indikator für eine Infektion sein. Mittels eines Temperatursensors wird beispielsweise eine Temperatur erfasst. Ein Anstieg der Temperatur bzw. eine erhöhte Temperatur kann ebenfalls ein Indikator für eine Infektion sein.

Der Beschleunigungssensor erfasst beispielsweise eine Bewegung oder eine Beschleunigung. Beispielsweise werden die von dem Beschleunigungssensor erfassten Daten herangezogen, um eine Elektronik der Gelenkendoprothese in einem inaktiven und in einem aktiven Modus zu betreiben, wobei die Elektronik zwischen dem inaktiven Modus und dem aktiven Modus in Abhängigkeit von mittels einem Beschleunigungssensor erfassten Daten umschaltbar ist. Basierend auf dem Erfassen einer Bewegung/Beschleunigung ist beispielweise der aktive Modus aktivierbar. In dem aktiven Modus wird beispielsweise mittels des Drucksensors und gegebenenfalls mittels weiteren vorhanden Sensoren, beispielsweise CRP-Sensor, Temperatursensor und ggf.

weiteren Drucksensoren, Daten erfasst und gegebenenfalls mittels der Datenübertragungseinrichtung übertragen. Mit dem Beschleunigungssensor können beispielsweise auch sehr kleine, kaum sichtbare, Bewegungen, auch als Mikrobewegung bezeichnet, erfasst werden. Solche Mikrobewegungen lassen beispielsweise auf eine ungewollte Lockerung der Gelenkendoprothese schließen.

Wenn keine Bewegung/Beschleunigung detektiert wird, beispielsweise über eine definierte Zeitdauer, ist beispielsweise der inaktive Modus aktivierbar. In dem inaktiven Modus werden mittels des Drucksensors und gegebenenfalls mittels weiteren vorhanden Sensoren, beispielsweise CRP-Sensor, Temperatursensor und ggf. weiteren Drucksensoren, keine Daten erfasst und gegebenenfalls auch keine Daten übertragen.

Es kann vorgesehen sein, dass die Gelenkendoprothese einen in eine Knochenkavität einführbaren Schaft umfasst, wobei die Energieversorgungseinrichtung oder Teile der Energieversorgungseinrichtung, und/oder die elektronischen Mittel oder Teile der elektronischen Mittel in dem Schaft angeordnet sind.

Weitere Ausführungsformen betreffen ein Verfahren zum Erfassen einer an einer Gleitfläche einer Gelenkendoprothese wirkenden Kraft, insbesondere eine Druckkraft, mittels einer Gelenkendoprothese gemäß den beschriebenen Ausführungsformen. Das Verfahren sieht vor, dass mittels einer Sensoreinrichtung, insbesondere Drucksensor, eine an einer Gleitfläche der Gelenkendoprothese wirkende Kraft, insbesondere eine Druckkraft, erfasst wird, wobei die erfasstem Daten mittels elektronischen Mittel von der mindestens einen Sensoreinrichtung Erhalten werden, wobei die Sensoreinrichtung und die elektronischen Mittel mittels einer Energieversorgungseinrichtung versorgt werden, und wobei die Sensoreinrichtung in die Gelenkendoprothese, insbesondere in Kunststoff, insbesondere Polyetheretherketon (PEEK) oder Polyetheretherketon, (PEEK),-Verbundwerkstoff, eingebettet ist.

Die Daten können zu Forschungs- und Wissenschaftszwecken und für die Weiterentwicklung bereitgestellt werden. Die Daten über die wirkenden Kräfte und gegebenenfalls weitere Daten können zur Weiterentwicklung und zur Ursachenforschung, beispielweise bei Fehlfunktion oder Bruch von Endoprothesen, herangezogen werden.

Eine Ausführungsform des Verfahrens sieht vor, dass die erfassten Daten mittels einer Datenübertragungseinrichtung übertragen werden, und dass basierend auf den übertragenen Daten mittels eine Anzeigeeinrichtung anzeigbare Informationen bereitgestellt werden. Die anzeigbare Informationen können beispielsweise auch einem Patienten Informationen bereitstellen.

Eine Ausführungsform des Verfahrens sieht vor, dass eine Elektronik der Gelenkendoprothese in einem inaktiven und in einem aktiven Modus betreibbar ist, und dass zwischen dem inaktiven Modus und dem aktiven Modus in Abhängigkeit von mittels einem Beschleunigungssensor erfassten Daten umgeschaltet wird. Basierend auf dem Erfassen einer Bewegung/Beschleunigung kann der aktive Modus aktiviert werden. In dem aktiven Modus wird beispielsweise mittels des Drucksensors und gegebenenfalls mittels weiteren vorhanden Sensoren, beispielsweise CRP-Sensor, Temperatursensor und ggf. weiteren Drucksensoren, Daten erfasst und gegebenenfalls mittels der Datenübertragungseinrichtung übertragen.

Wenn keine Bewegung/Beschleunigung detektiert wird, beispielsweise über eine definierte Zeitdauer, wird der inaktive Modus aktiviert. In dem inaktiven Modus werden mittels des Drucksensors und gegebenenfalls mittels weiteren vorhanden Sensoren, beispielsweise CRP-Sensor, Temperatursensor und ggf. weiteren Drucksensoren, keine Daten erfasst und gegebenenfalls auch keine Daten übertragen.

Weitere Ausführungsformen betreffen ein System umfassend eine Gelenkendoprothese gemäß den beschriebenen Ausführungsformen, und eine Anzeigeeinrichtung zum Anzeigen von Informationen basierend auf mit der Gelenkendoprothese erfassten Daten. Die Anzeigeeinrichtung ist beispielsweise ein Display eines Mobilfunkgeräts, das Daten von der Gelenkendoprothese empfängt.

Weitere Ausführungsformen betreffen ein Computerprogrammprodukt mit einem Computerprogramm, welches durch Ausführen in einer Recheneinrichtung eine Anzeigeeinrichtung eines Systems, dazu veranlasst, Informationen basierend auf mit der Gelenkendoprothese erfassten Daten anzuzeigen. Die Recheneinrichtung ist beispielsweise ein Prozessor eines Mobilfunkgeräts, das Daten von der Gelenkendoprothese empfängt.

Weitere Ausführungsformen betreffen ein Herstellungsverfahren zum Herstellen einer Gelenkendoprothese, insbesondere Knieendoprothese, beispielsweise eine Femurkomponente oder eine Tibiakomponente gemäß den vorstehend beschriebenen Ausführungsformen. Gemäß dem Verfahren ist vorgesehen, dass die Herstellung ein Spritzgussverfahren oder ein 3D-Druckverfahren umfasst, wobei wenigstens eine Sensoreinrichtung in einem Spritzgussverfahren mit einem Kunststoff, beispielweise Polyetheretherketon (PEEK) oder Polyetheretherketon, (PEEK),-Verbundwerkstoff, umspritzt wird oder in einem 3D-Druckverfahren in den Kunststoff, beispielweise Polyetheretherketon (PEEK) oder Polyetheretherketon, (PEEK),-Verbundwerkstoff, eingebettet wird. Die Einbettung der Sensoreinrichtung erfolgt also vorteilhafterweise direkt im formgebenden Herstellungsschritt der Gelenkendoprothese. Die Sensoreinrichtung wird insbesondere nicht nachträglich nach dem formgebenden Herstellungsschritt in die Gelenkendoprothese eingebettet oder an der Gelenkendoprothese montiert.

Weitere Vorteile ergeben sich aus der Beschreibung und den beigefügten Zeichnungen. Ausführungsbeispiele der Erfindung sind in den Zeichnungen dargestellt und werden in der nachfolgenden Beschreibung näher erläutert. Dabei bezeichnen gleiche Bezugszeichen in verschiedenen Figuren jeweils gleiche oder zumindest ihrer Funktion nach vergleichbare Elemente. Bei der Beschreibung einzelner Figuren wird gegebenenfalls auch auf Elemente aus anderen Figuren Bezug genommen. Es zeigen jeweils in schematischer Form:
- Fig. 1: eine erfindungsgemäße Gelenkendoprothese gemäß einer ersten Ausführungsform in drei verschiedenen Ansichten (a, b, c);
- Fig. 2: einen Gelenkkörper bildenden Teil der erfindungsgemäßen Gelenkendoprothese aus Fig. 1 in zwei verschiedenen Ansichten (a, b);
- Fig. 3: elektronische Komponenten der Gelenkendoprothese;
- Fig. 4: einen Teil der elektronischen Komponenten aus Fig. 3 der Gelenkendoprothese in einer Explosionsdarstellung;
- Fig. 5: eine erfindungsgemäße Gelenkendoprothese gemäß einer zweiten Ausführungsform in drei verschiedenen Ansichten (a, b);
- Fig. 6: eine erfindungsgemäße Gelenkendoprothese gemäß einer dritten Ausführungsform in zwei verschiedenen Ansichten (a, b);
- Fig. 7: elektronische Komponenten der Gelenkendoprothese aus Fig. 6, und
- Fig. 8: eine Anzeigeeinrichtung.

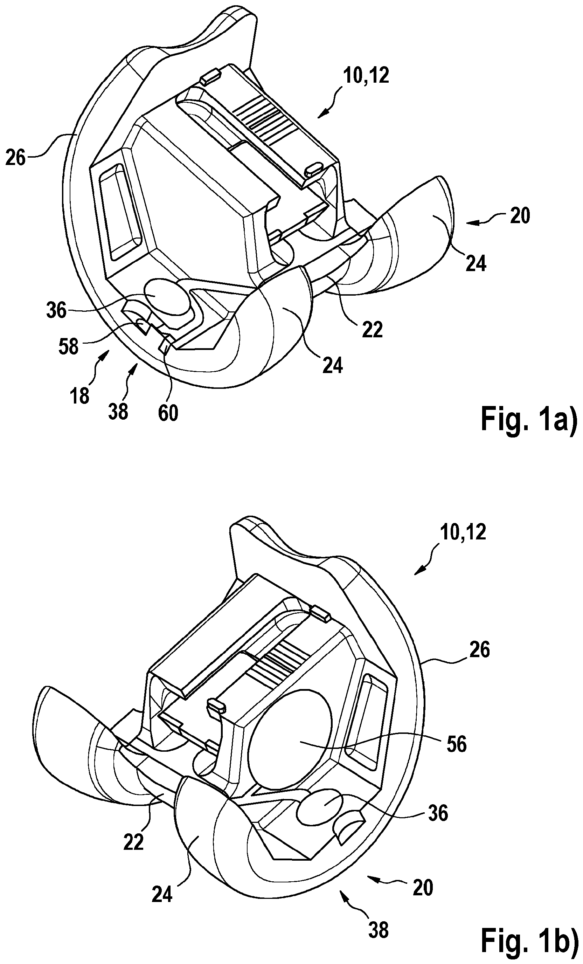

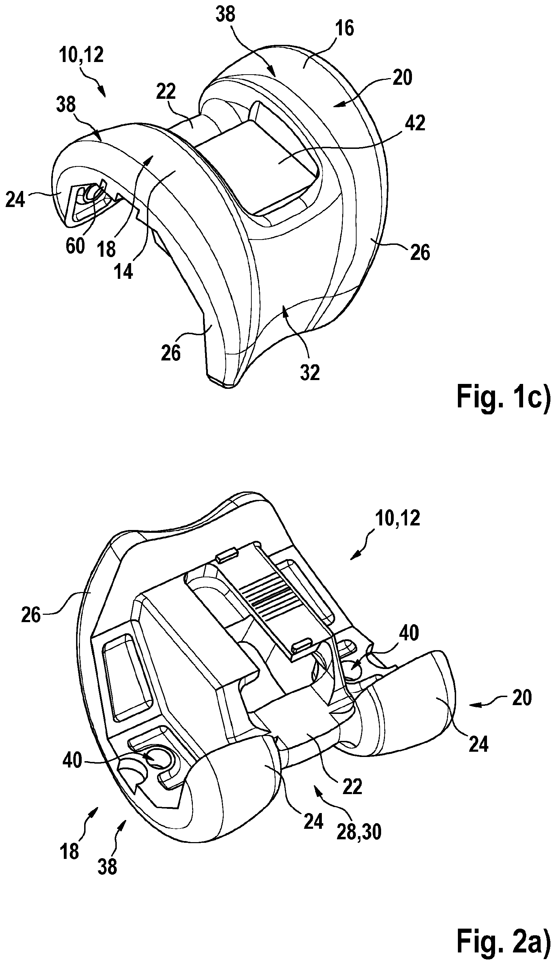

Figur 1 zeigt eine erfindungsgemäße Gelenkendoprothese. Die Gelenkendoprothese ist in ihrer Gesamtheit mit dem Bezugszeichen 10 versehen. Im Beispiel ist die Gelenkendoprothese 10 eine Knieendoprothese. Die Knieendoprothese 10 umfasst im Beispiel eine an einem Femurknochen verankerbare Femurkomponente 12. Die Knieendoprothese 10 kann auch eine Tibiakomponente und eine, insbesondere an die Tibiakomponente angeformte Meniskuskomponente umfassen (in den Figuren nicht dargestellt).

Die Femurkomponente 12 umfasst im Beispiel eine mediale und eine lateralen Femurgelenkfläche 14 und 16, welche äußere Flächen zweier Femurkondylen 18 und 20, nämlich einer medialen Kondyle 18 sowie einer lateralen Kondyle 20, der Femurkomponente 12 bilden. Die Femurgelenkflächen 14 und 16 sind korrespondierend oder im Wesentlichen korrespondierend ausgebildet zum Zusammenwirken mit Meniskusgelenkflächen der Meniskuskomponente.

Es kann vorteilhaft sein, wenn die Femurgelenkflächen 14 und 16 jeweils einen Ausschnitt einer Kugeloberfläche bilden. Die Femurgelenkflächen 14 und 16 sind zusammen mit den Meniskusgelenkflächen derart ausgebildet, dass die jeweils aneinander liegenden Gelenkflächen aneinander abgleiten und/oder abrollen.

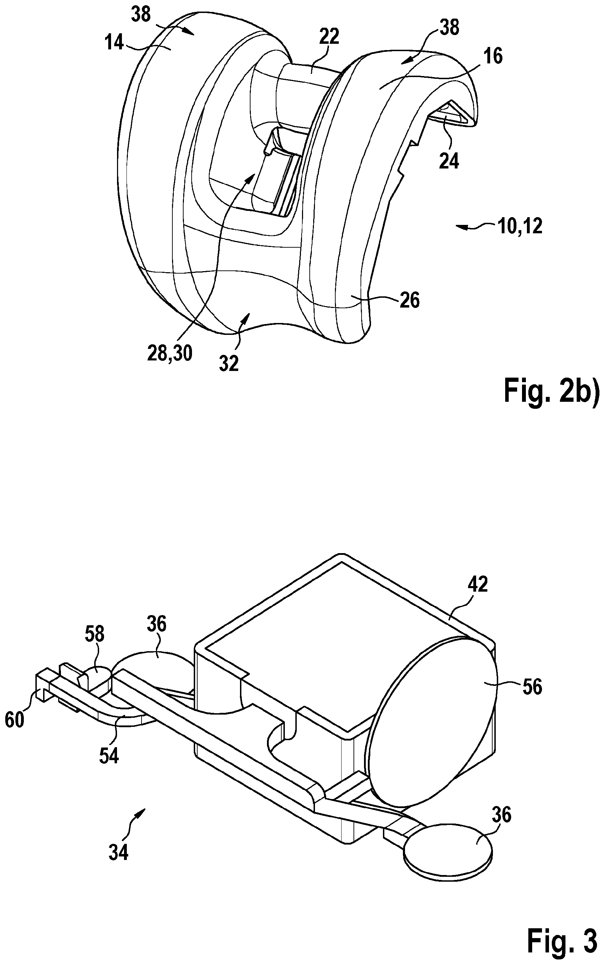

Im Beispiel ein Stabilisierungselement 22 zwischen posterioren Enden 24 der Kondylen 18 und 20 angeordnet und verbindet diese in lateral-medialer Richtung miteinander. Anteriore Enden 26 der Kondylen 18 und 20 sind direkt miteinander verbunden. Insgesamt wird so eine Ausnehmung 28 in Form einer Durchbrechung 30 definiert, welche seitlich und anterior durch die Kondylen 18 und 20 sowie posterior durch das Stabilisierungselement 22 begrenzt wird, vgl. Fig. 2.

Im Bereich der anterioren Enden 26 der Kondylen 18 und 20 ist ein trochlearer Bereich 32, auch trochleare Gleitrinne oder Trochlea genannt, ausgebildet.

Die Femurkomponente 12 an sich, also der die körperliche Form der Femurkomponente 12 bildende Teil, ist im Beispiel aus Kunststoff, beispielweise Polyetheretherketon (PEEK) oder Polyetheretherketon, (PEEK),-Verbundwerkstoff, gefertigt. Die Herstellung kann beispielsweise in einem Spritzgussverfahren oder einem 3D-Druckverfahren erfolgen.

Die Gelenkendoprothese 10 umfasst im Beispiel elektronische Komponenten 34. In Fig. 3 sind die elektronischen Komponenten 34 separat dargestellt. In Fig. 1 sind die elektronischen Komponenten 32 an bzw. teilweise auch in der Gelenkendoprothese 10 angeordnet.

Gemäß den Figuren 1 und 3 umfassen die elektronische Komponenten 34 zwei Sensoreinrichtungen 36 zum Erfassen von an Gleitflächen der Gelenkendoprothese 10 wirkenden Kräften, insbesondere Druckkräfte. Die Sensoreinrichtungen 36 werden vereinfacht auch als Drucksensoren 36 bezeichnet. Im Beispiel ist ein jeweiliger Drucksensor 36 in einem distalen Bereich 38 Kondylen 18 und 20 angeordnet. In dem distalen Bereich 38 der Kondylen wirken bei einem Patienten in einem stehenden Zustand und bei einer Laufbewegung relativ große Kräfte an den Femurgelenkflächen 14 und 16.

Im Beispiel sind die Drucksensoren 36 bei der Gelenkendoprothese 10 in entsprechende Aussparungen 40 eingebettet. Die Aussparungen 40 sind beispielsweise auch in Fig. 2 dargestellt, die die Gelenkendoprothese 10 ohne die elektronischen Komponenten 34 zeigt.

Die elektronischen Komponenten 34 umfassen weiter elektronische Mittel zum Erhalten der von den Drucksensoren 36 erfassten Daten und sowie eine Energieversorgungseinrichtung zum Versorgen der Drucksensoren und der elektronischen Mittel.

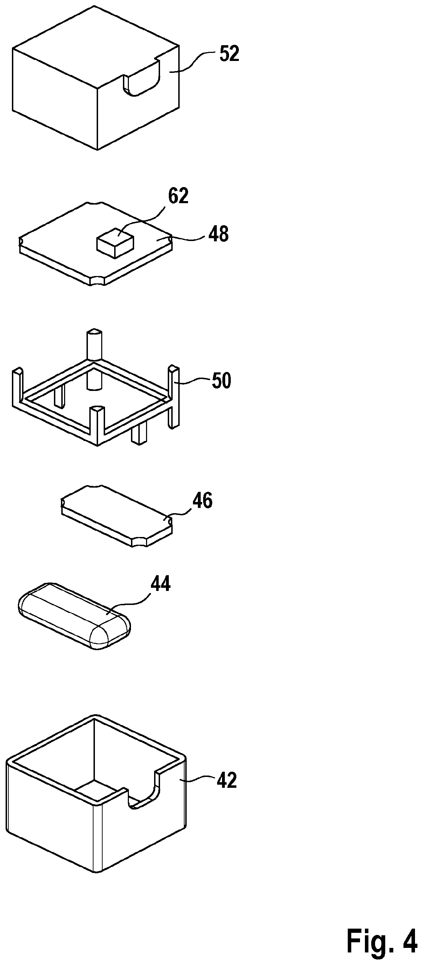

Im Beispiel ist ein Teil der elektronischen Komponenten in einem Gehäuse 42 angeordnet, vgl. Fig. 3 und 4. In dem Gehäuse ist ein Energiespeicher 44, zwei Leiterplatten 46, 48, insbesondere mit weitere nicht im Detail dargestellter Elektronik, Trägerelemente 50 zusammen mit Vergussmasse 52 angeordnet. In dem Gehäuse 42 ist beispielsweise auch ein Datenspeicher, insbesondere zum Speichern und/oder Zwischenspeichern der von den Drucksensoren 36 erfassten Daten, sowie eine Datenübertragungseinrichtung zum Übertragen der gespeicherten oder der erfassten Daten vorgesehen. Die Datenübertragungseinrichtung ist dazu ausgebildet, Daten drahtlos, insbesondere an eine externe Empfangseinrichtung zu übertragen. Die drahtlose Datenübertragung erfolgt beispielweise mittels einer Bluetooth basierten oder einer NFC basierten Datenübertragung. Die Datenübertragung erfolgt beispielsweise an ein Mobilfunkgerät.

Gemäß dem Beispiel sind die Drucksensoren 36 mit entsprechenden elektronischen Verbindungsmitteln 54 mit der Elektronik in dem Gehäuse 42, insbesondere mit dem Energiespeicher, dem Datenspeicher und/oder der Datenübertragungseinrichtung verbunden. Über die Verbindungsmittel 54 erfolgt beispielweise eine Energieversorgung aus dem Energiespeicher 44 und/oder eine Datenübertragung zwischen Drucksensoren 36 und dem Datenspeicher und/oder der Datenübertragungseinrichtung.

Im Beispiel sind neben den Drucksensoren 36 weitere der elektronischen Komponenten, beispielsweise ein Teil der elektronischen Verbindungsmittel 54 bei der Gelenkendoprothese 10 in entsprechende Aussparungen eingebettet. Die Aussparungen werden beispielsweise beim Herstellen der Gelenkendoprothese, beispielsweise mittel Spritzguss und/oder 3D-Druck, vorgesehen. Alternativ können die entsprechenden elektronischen Komponenten bereits beim Spritzgießen der Gelenkendoprothese 10 in die Gelenkendoprothese 10 integriert, sozusagen eingegossen, werden. Ferner umfasst die Gelenkendoprothese 10 eine Aussparung für das Gehäuse 42, im Bereich eines nicht dargestellten Schafts.

Gemäß dem Beispiel umfasst die Gelenkendoprothese 10 eine Empfangsspule 56 zum Aufladen des Energiespeichers 44 mittels induktivem Laden. In die Empfangsspule ist mittels einer außerhalb des Körpers des Patienten in die Nähe bringbare Sendespule eine Spannung induzierbar. Auf diese Weise kann der Energiespeicher 44, insbesondere bedarfsweise geladen werden.

Gemäß dem Beispiel umfassen die elektronischen Komponenten 34 der Gelenkendoprothese 10 weitere Sensorik.

Beispielweise sind Sensoren vorgesehen, die eine frühzeitige Erkennung von Infektionen ermöglichen. Mittels eines CRP-Sensors 58 kann beispielsweise das Vorhandensein bzw. eine Konzentration von C-reaktiven Protein erfasst werden. Mittels eines Temperatursensors 60 wird beispielsweise eine Temperatur erfasst. Ein Anstieg der Temperatur bzw. eine erhöhte Temperatur kann ebenfalls ein Indikator für eine Infektion sein.

In den Figuren nicht dargestellt, können auch weitere Drucksensoren vorgesehen werden. Beispielsweise kann ein Drucksensor oder mehrere Drucksensoren in einem Bereich der posterioren Enden 24, 26 der Kondylen 18, 20 vorgesehen werden. In diesem Bereich wirken bei einem Patienten beim Beugen der Kniegelenke, beispielsweise beim Treppensteigen, relativ große Kräfte an den Femurgelenkflächen 12, 16. Beispielsweise können ein oder mehrere Drucksensoren im Bereich der Trochlea 32 vorgesehen werden. In diesem Bereich kann eine Kraft gemessen werden, die an der Fläche zwischen Trochlea und Patella wirkt, auch Patelladruck genannt. Werte des Patalladrucks können ein Indikator für eine korrekte oder inkorrekte Positionierung der Endoprothese sein.

Im Beispiel umfasst die Gelenkendoprothese 10 einen Beschleunigungssensor 62. Der Beschleunigungssensor 62 erfasst beispielsweise eine Bewegung oder eine Beschleunigung. Beispielsweise werden die von dem Beschleunigungssensor 62 erfassten Daten herangezogen, um eine Elektronik der Gelenkendoprothese in einem inaktiven und in einem aktiven Modus zu betreiben, wobei die Elektronik zwischen dem inaktiven Modus und dem aktiven Modus in Abhängigkeit von mittels einem Beschleunigungssensor erfassten Daten umschaltbar ist.

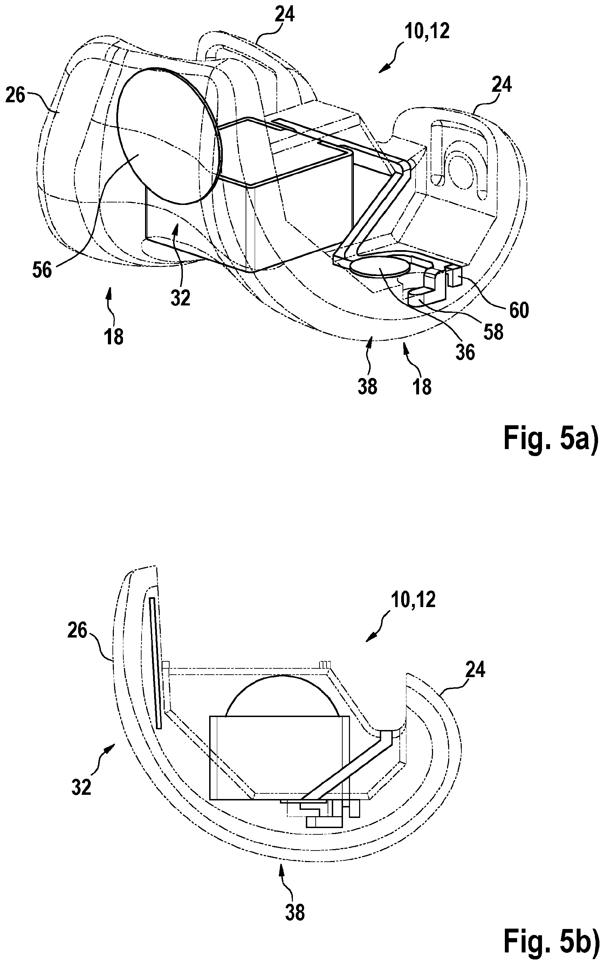

Die Figur 5 zeigt eine Gelenkendoprothese 10 gemäß einer weiteren Ausführungsform. Die Empfangsspule 56 ist im Bereich der Trochlea 32 angeordnet.

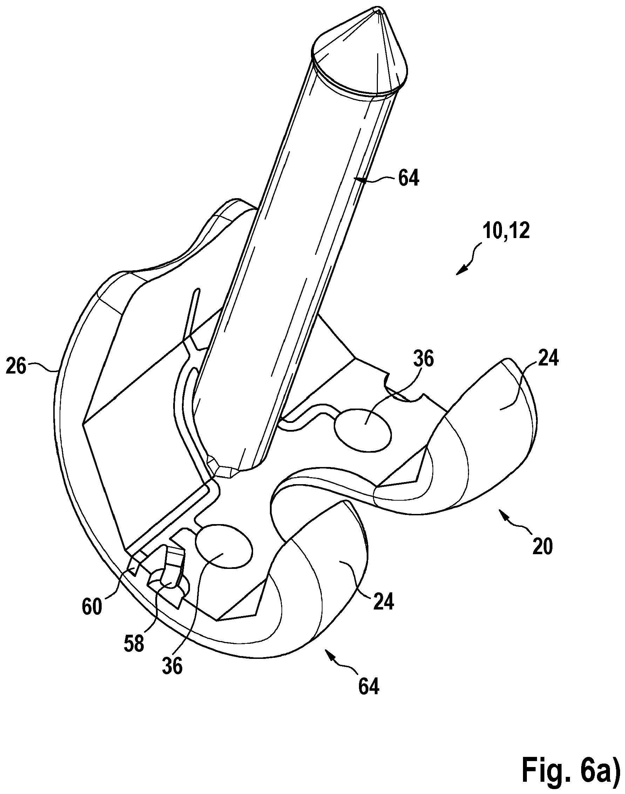

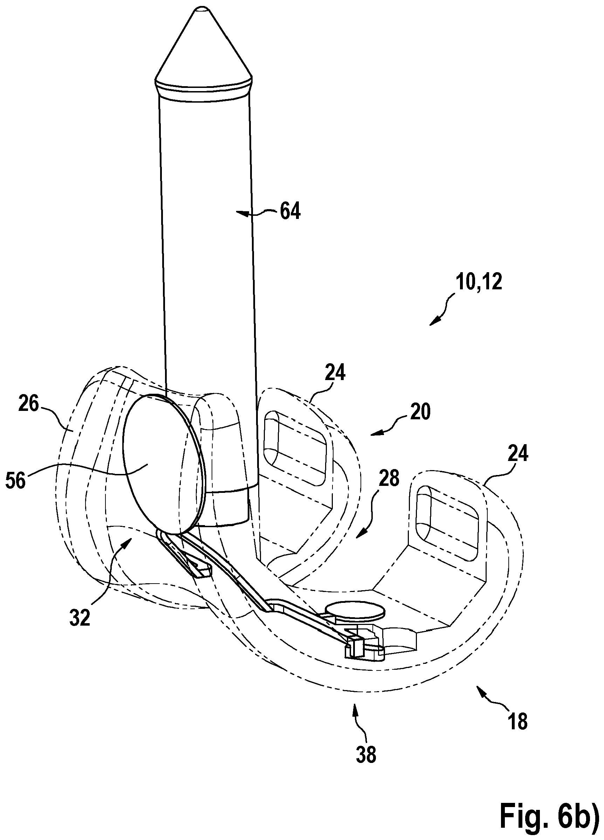

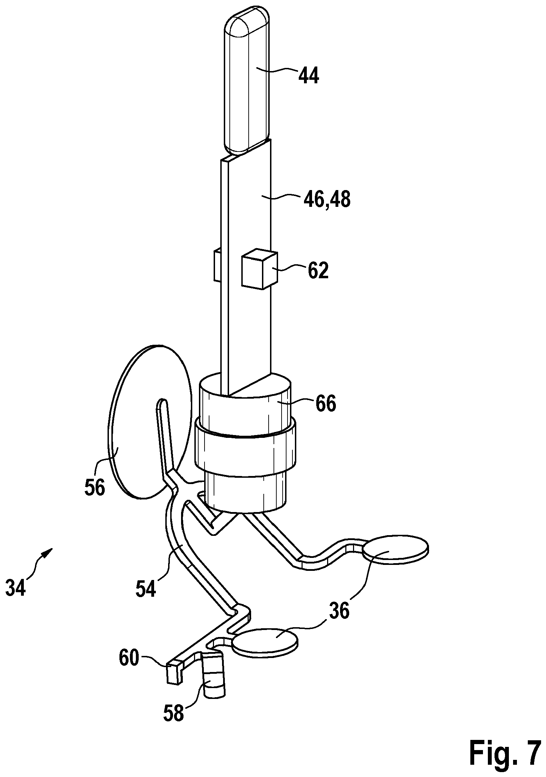

Die Figur 6 zeigt eine Gelenkendoprothese 10 gemäß einer weiteren Ausführungsform. Die Gelenkendoprothese 10 umfasst einen in eine Knochenkavität, im Beispiel in einen Femurknochen, einführbaren Schaft 64. Im Beispiel ist ein Teil der elektronischen Komponenten 34 in dem Schaft 64 angeordnet. Figur 7 zeigt die elektronischen Komponenten ohne den Schaft 64. Mittels eines Adapterteils 66 kann der im Schaft angeordnete Teil der elektronischen Komponenten mit dem außerhalb des Schafts angeordneten Teil der elektronischen Komponenten gekoppelt werden. Der Schaft 64 bildet ein Gehäuse für die darin angeordnete Elektronik, vgl. Fig. 6.

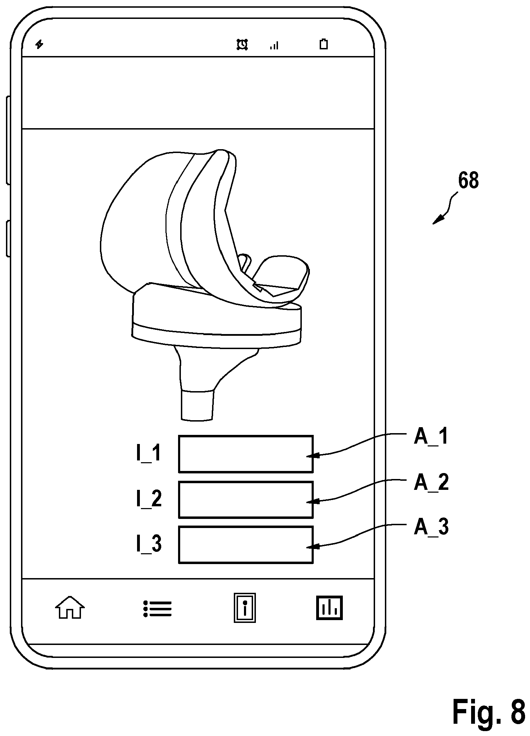

Figur 8 zeigt eine Anzeigeeinrichtung 68. Die Anzeigeeinrichtung 68 ist beispielsweise ein Mobilfunkgerät. Die Anzeigeeinrichtung 68 empfängt beispielsweise Daten von der Datenübertragungseinrichtung der Gelenkendoprothese 10. Mittels einer geeigneten Applikation, App, werden basierend auf den empfangenen Daten auf einem Display der Anzeigeeinrichtung 68 Informationen dargestellt. In insgesamt drei Anzeigefelder wird beispielsweise drei verschiedene Informationen I_1, I_2 und I_3 dargestellt. In einem ersten Anzeigefeld A_1 wird beispielsweise eine Information über eine Temperatur, die mittels des Temperatursensors 60 der Gelenkendoprothese 10 erfasst wird, dargestellt. In einem zweiten Anzeigefeld A_2 wird beispielsweise eine Information über Kraft bzw. Druckkraft, die mittels der Drucksensors 36 der Gelenkendoprothese 10 erfasst wird, dargestellt. In einem dritten Anzeigefeld A_3 wird beispielsweise eine Information über einen CRP-Konzentration, die mittels des CRP-Sensors 58 der Gelenkendoprothese 10 erfasst wird, dargestellt. Die Informationen können als absolute Werte oder in Abhängigkeit von entsprechenden Grenzwerten als in Ordnung (OK), kritisch oder nicht Ordnung (nicht OK), gegebenenfalls in Kombination mit Hinweisen und/oder Farben, beispielweise grün, orangen, rot, angezeigt werden. Die Applikation kann beispielsweise bei der Rehabilitation bzw. Genesung unterstützen. Beispielweise kann über die Anzeige der Informationen der Patient über die aktuelle Belastung des Gelenks informiert werden. Die Anwendung kann auch kombiniert werden mit Informationen über zulässige Belastungen und Beugungswinkel. Der Patient kann durch Anwendung dieser Informationen den Umgang mit der Gelenkendoprothese 10 erlernen und verbessern.

## Patentansprüche

1. Gelenkendoprothese (10), insbesondere Knieendoprothese (10), wobei die Gelenkendoprothese (10) zumindest teilweise aus einem Kunststoff, insbesondere Polyetheretherketon (PEEK) oder Polyetheretherketon, (PEEK),-Verbundwerkstoff, gefertigt ist, oder Kunststoff, insbesondere Polyetheretherketon (PEEK) oder Polyetheretherketon, (PEEK),-Verbundwerkstoff, umfasst, wobei die Gelenkendoprothese (10) wenigstens eine Sensoreinrichtung (36), insbesondere Drucksensor (36), zum Erfassen einer an einer Gleitfläche der Gelenkendoprothese (10) wirkenden Kraft, insbesondere eine Druckkraft, elektronische Mittel (34, 54) zum Erhalten der von der mindestens einen Sensoreinrichtung (36) erfassten Daten und eine Energieversorgungseinrichtung (44, 56) zum Versorgen der wenigstens einen Sensoreinrichtung (36) und der elektronischen Mittel (34, 54) umfasst, und wobei die wenigstens eine Sensoreinrichtung (36) in die Gelenkendoprothese (10), insbesondere in Kunststoff, insbesondere Polyetheretherketon (PEEK) oder Polyetheretherketon, (PEEK),-Verbundwerkstoff, eingebettet ist.

2. Gelenkendoprothese (10) nach Anspruch 1, wobei die Energieversorgungseinrichtung (44, 56) oder Teile der Energieversorgungseinrichtung (44, 56), und/oder die elektronischen Mittel (34, 54) oder Teile der elektronischen Mittel (34, 54) in der Gelenkendoprothese (10), insbesondere in Kunststoff, insbesondere Polyetheretherketon (PEEK) oder Polyetheretherketon, (PEEK),-Verbundwerkstoff, eingebettet sind.

3. Gelenkendoprothese (10) nach einem der Ansprüche 1 oder 2, wobei die Energieversorgungseinrichtung (44, 56) einen Energiespeicher (44) und Mittel (56) zum Aufladen des Energiespeichers (44), insbesondere Mittel zum induktiven Laden, umfasst.

4. Gelenkendoprothese (10) nach einem der vorhergehenden Ansprüche, wobei die Gelenkendoprothese (10), insbesondere die elektronischen Mittel (34) der Gelenkendoprothese (10), eine Datenübertragungseinrichtung umfasst, wobei die Datenübertragungseinrichtung ausgebildet ist, Daten drahtlos, insbesondere an eine externe Empfangseinrichtung zu übertragen.

5. Gelenkendoprothese (10) nach einem der vorhergehenden Ansprüche, wobei die Gelenkendoprothese (10) eine Kniegelenkendoprothese (10) ist oder zumindest einen Teil einer Kniegelenkendoprothese (10) bildet, wobei die Gelenkendoprothese (10) wenigstens eine erste Gelenkkomponente (12), beispielsweise eine Femurkomponente (12) oder eine Tibiakomponente oder eine Meniskuskomponente, ist oder umfasst.

6. Gelenkendoprothese (10) nach Anspruch 5, wobei die erste Gelenkkomponente, eine Femurkomponente (12) ist, und die Femurkomponente (12) wenigstens eine Femurkondyle (18, 20) umfasst, wobei wenigstens eine Sensoreinrichtung (36) zum Erfassen einer an einer Gleitfläche einer Gelenkendoprothese (10) wirkenden Kraft in einem distalen Bereich (38) der Femurkondyle (18, 20) vorgesehen ist, wobei die Femurkondyle (18, 20) eine Femurgelenkfläche (14, 16) umfasst, wobei die Femurgelenkfläche (14, 16) eine Gleitfläche der Gelenkendoprothese (10) bildet und mittels der Sensoreinrichtung (36) die an der Femurgelenkfläche (14, 16) in dem distalen Bereich (38) der Femurkondyle (18, 20) wirkende Kraft erfassbar ist.

7. Gelenkendoprothese (10) nach einem der Ansprüche 5 oder 6, wobei die erste Gelenkkomponente, eine Femurkomponente (12) ist, und die Femurkomponente (12) wenigstens eine retropatellare Gleitrinne, Trochlea (32), umfasst, und wobei wenigstens ein Teil der Energieversorgungseinrichtung (44, 56) im Bereich der Trochlea (32) vorgesehen ist.

8. Gelenkendoprothese (10) nach einem der Ansprüche 5 bis 7, wobei die erste Gelenkkomponente, eine Femurkomponente (12) ist, und die Femurkomponente (12) wenigstens eine Femurkondyle (18, 20) und/oder Trochlea umfasst, und wobei eine Sensoreinrichtung (36) oder eine weitere Sensoreinrichtung (36) zum Erfassen einer an einer Gleitfläche der Gelenkendoprothese (10) wirkenden Kraft in einem posterioren Bereich (24) der Femurkondyle (18, 20) vorgesehen ist, und/oder wobei eine Sensoreinrichtung (36) oder eine weitere Sensoreinrichtung (36) zum Erfassen einer an einer Gleitfläche der Gelenkendoprothese (10) wirkenden Kraft im Bereich der Trochlea (32) vorgesehen ist.

9. Gelenkendoprothese (10) nach einem der vorhergehenden Ansprüche, wobei die Gelenkendoprothese (10) wenigstens eine Sensoreinrichtung (58) zum Detektieren einer Infektion in der Gelenkendoprothese (10) und/oder in einem Implantationsbereich der Gelenkendoprothese (10), insbesondere mittels Erfassen von C-Reaktivem Protein, und/oder wenigstens eine Sensoreinrichtung (60) zum Erfassen einer Temperatur, und/oder wenigstens eine Sensoreinrichtung (62) zum Erfassen einer Beschleunigung umfasst.

10. Gelenkendoprothese (10) nach einem der vorhergehenden Ansprüche, wobei die Gelenkendoprothese (10) einen in eine Knochenkavität einführbaren Schaft (64) umfasst, wobei die Energieversorgungseinrichtung (44, 56) oder Teile der Energieversorgungseinrichtung (44, 56), und/oder die elektronischen Mittel (34, 54) oder Teile der elektronischen Mittel (34, 54) in dem Schaft (68) angeordnet sind.

11. Verfahren zum Erfassen einer an einer Gleitfläche einer Gelenkendoprothese (10) wirkenden Kraft, insbesondere eine Druckkraft, mittels einer Gelenkendoprothese (10) nach einem der Ansprüche 1 bis 10, wobei mittels eines Sensoreinrichtung (36), insbesondere Drucksensor (36), eine an einer Gleitfläche der Gelenkendoprothese (10) wirkende Kraft, insbesondere eine Druckkraft, erfasst wird, wobei die erfasstem Daten mittels elektronischen Mitteln (34, 54) von der mindestens einen Sensoreinrichtung (36) Erhalten werden, wobei die Sensoreinrichtung (36) und die elektronischen Mittel (34, 54) mittels einer Energieversorgungseinrichtung (44, 56) versorgt werden, und wobei die Sensoreinrichtung (36) in die Gelenkendoprothese (10), insbesondere in Kunststoff, insbesondere Polyetheretherketon (PEEK) oder Polyetheretherketon, (PEEK),-Verbundwerkstoff, eingebettet ist.

12. Verfahren nach Anspruch 11, wobei die erfassten Daten mittels einer Datenübertragungseinrichtung übertragen werden, und wobei basierend auf den übertragenen Daten mittels einer Anzeigeeinrichtung (68) anzeigbare Informationen bereitgestellt werden.

13. Verfahren nach einem der Ansprüche 11 oder 12, wobei eine Elektronik (34) der Gelenkendoprothese (10) in einem inaktiven und in einem aktiven Modus betreibbar ist, und wobei zwischen dem inaktiven Modus und dem aktiven Modus in Abhängigkeit von mittels einem Beschleunigungssensor (62) erfassten Daten umgeschaltet wird.

14. System umfassend eine Gelenkendoprothese (10) nach einem der Ansprüche 1 bis 10, und eine Anzeigeeinrichtung (68) zum Anzeigen von Informationen basierend auf mit der Gelenkendoprothese (10) erfassten Daten.

15. Computerprogrammprodukt mit einem Computerprogramm, welches durch Ausführen in einer Recheneinrichtung eine Anzeigeeinrichtung (68) eines Systems nach Anspruch 14, dazu veranlasst, Informationen basierend auf mit der Gelenkendoprothese (10) erfassten Daten anzuzeigen.
